Europäisches Patentamt

European Patent Office

Office européen des brevets

(11) Numéro de publication: **0 095 971**
A1

## (12) DEMANDE DE BREVET EUROPEEN

(21) Numéro de dépôt: **83401014.2**

(22) Date de dépôt: **20.05.83**

(51) Int. Cl.³: **A 61 L 2/20,** A 61 L 2/06

(30) Priorité: **28.05.82 FR 8209371**

(43) Date de publication de la demande: **07.12.83**
**Bulletin 83/49**

(84) Etats contractants désignés: **BE CH DE GB IT LI**

(71) Demandeur: **LA CALHENE Société Anonyme, 5, rue Emile Zola, F-95870 Bezons (FR)**

(72) Inventeur: **Saint Martin, Bernard, 6, rue Leclerc, F-75014 Paris (FR)**
Inventeur: **Picard, Claude, 34, rue Zilina, F-92000 Nanterre (FR)**

(74) Mandataire: **Mongrédien, André et al, c/o BREVATOME 25, rue de Ponthieu, F-75008 Paris (FR)**

(54) **Procédé et installation pour la stérilisation, l'introduction dans un isolateur et l'extraction d'un produit en vrac.**

(57) L'invention concerne un procédé et une installation pour stériliser, introduire dans un isolateur stérile ou pathogène (10) et enfin extraire à volonté un produit (P) en vrac.

La stérilisation et l'introduction dans l'isolateur peuvent se faire soit en continu, soit de façon discontinue, au moyen d'une enceinte de stérilisation (26) ouvrant directement dans l'isolateur et apte à être séparée de ce dernier au moyen d'une porte (30). L'isolateur est équipé d'un circuit de ventilation-filtration (12), de moyens de stérilisation et de moyens de manipulation (32 à 36). On peut extraire le produit en continu par un sas liquide ou de façon discontinue au moyen de l'enceinte de stérilisation (26) ou d'un conteneur (38) apte à être accouplé à l'isolateur par un système de transfert à double porte (40).

Application à la pharmacie, la recherche biologique, la médecine et l'industrie.

La présente invention se rapporte à un procédé et à une installation pour stériliser, introduire dans un isolateur et enfin extraire à volonté un produit ou un matériel quelconque sans interrompre la chaîne stérile et sans nécessiter l'emballage du produit ou du matériel.

Dans certains domaines tels que la pharmacie, la recherche biologique, la médecine et l'industrie, on souhaite disposer d'une enceinte bactériologiquement étanche constituée par un isolateur qui doit à la fois protéger l'homme et l'environnement de toute contamination pouvant provenir de micro-organismes présents dans le volume clos et assurer le maintien rigoureux du caractère stérile de l'atmosphère contenue dans ce volume.

Le problème posé par cette "contamination croisée" se trouve compliqué par la nécessité d'introduire et d'extraire en cours d'expérimentation des produits et des matériels.

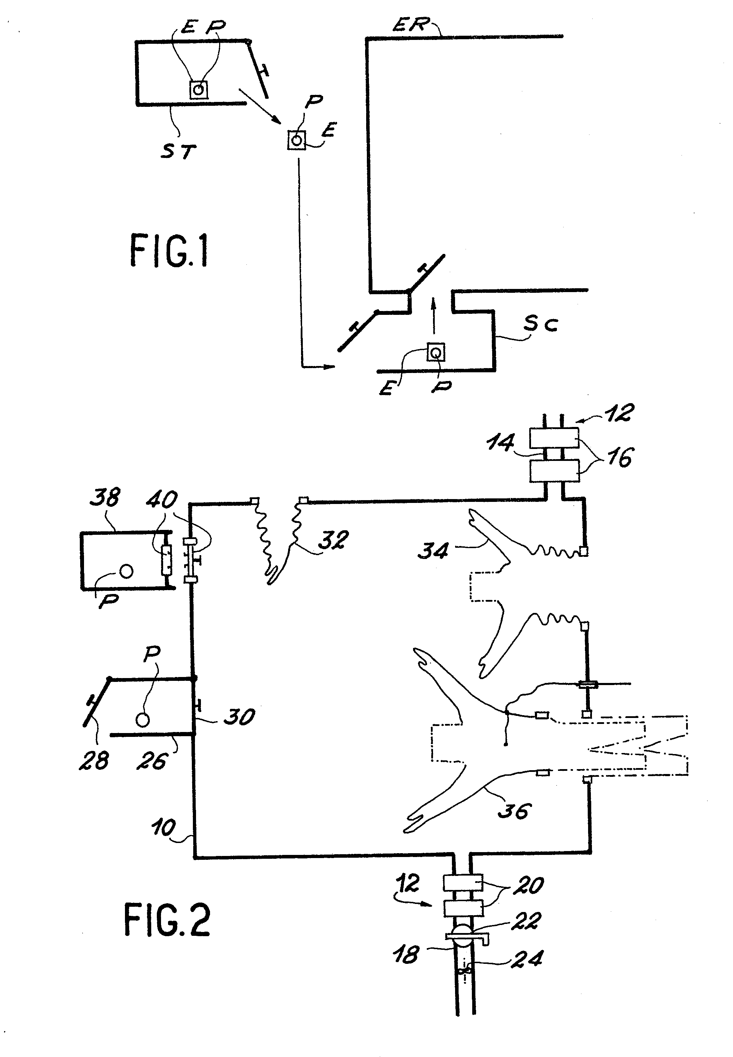

Actuellement, comme illustré schématiquement sur la figure 1, ce problème est résolu au moyen d'une enceinte de stérilisation ST totalement séparée de l'isolateur, ou enceinte de réception ER. La stérilisation des produits ou matériels P dans l'enceinte ST, s'effectue par des moyens thermiques (autoclave vapeur ou four type Pasteur) à l'intérieur d'un emballage E tel qu'une boîte en acier, ou qu'un sac en papier ou en plastique soudé. Cet emballage est nécessaire au maintien en atmosphère stérile des produits et des matériels lorsque ceux-ci sont transportés de l'enceinte de stérilisation ST jusqu'à l'enceinte de réception ER dans laquelle ils doivent être introduits. De plus, afin de ne pas contaminer cette dernière enceinte, il est nécessaire de stériliser à nou-

B 7531 GP

2 0095971

veau l'extérieur des emballages contenant les produits ou les matériels juste avant de les introduire dans l'enceinte. A cet effet, on utilise un stérilisateur chimique SC.

On voit que dans les installations actuelles, la chaîne nécessaire au maintien en atmosphère stérile des produits ou des matériels à introduire dans l'enceinte de réception est relativement longue, complexe et coûteuse. En particulier, la stérilisation dans une enceinte totalement distincte de l'enceinte de réception nécessite de stériliser les produits dans des emballages permettant leur transport et la présence même de ces emballages nécessite ensuite qu'ils soient eux-mêmes stérilisés avant d'être introduits dans l'enceinte de réception.

La présente invention a précisément pour objet un procédé et une installation de stérilisation et de stockage ne présentant pas les inconvénients de la technique antérieure et permettant notamment une stérilisation en vrac des produits et des matériels, ce qui supprime les emballages et, par conséquent, la nécessité de stériliser à nouveau l'extérieur de ces emballages avant de les introduire dans l'enceinte de réception. La chaîne stérile se trouve ainsi très simplifiée et par conséquent moins longue et moins coûteuse.

A cet effet, il est proposé conformément à une première variante de réalisation de l'invention un procédé pour la stérilisation, l'introduction dans une enceinte de stockage biologiquement étanche et l'extraction d'un produit en vrac, caractérisé en ce qu'il comprend les étapes successives suivantes :

- stérilisation initiale de l'enceinte de stockage,
- introduction du produit en vrac dans une enceinte de stérilisation par une porte ouvrant sur l'extérieur, ladite enceinte de stérilisation communiquant avec l'enceinte de stockage par une autre porte,

B 7531 GP

0095971

- fermeture des portes de l'enceinte de stérilisation,
- stérilisation du produit en vrac dans l'enceinte de stérilisation,
- ouverture de la porte par laquelle l'enceinte de stérilisation communique avec l'enceinte de stockage,
- introduction du produit stérile directement dans l'enceinte de stockage,
- stockage et manipulation du produit dans l'enceinte de stockage,
- extraction du produit en vrac hors de l'enceinte de stockage, en introduisant ce produit dans au moins un conteneur de transfert communiquant avec l'enceinte de stockage par un système de transfert à double porte après ouverture de la double porte, en fermant la double porte et en déconnectant le conteneur de l'enceinte de stockage.

Conformément à une deuxième variante de réalisation de l'invention, il est également proposé un procédé pour la stérilisation, l'introduction dans une enceinte de traitement biologiquement étanche et l'extraction en continu d'un produit en vrac, caractérisé en ce qu'il comprend les étapes successives suivantes :

- stérilisation initiale de l'enceinte de traitement après fermeture d'une porte par laquelle cette dernière communique avec une enceinte de stérilisation en forme de tunnel,
- mise en route de moyens de stérilisation à l'intérieur du tunnel,
- ouverture de ladite porte,
- introduction en continu du produit en vrac par une extrémité du tunnel ouvrant sur l'extérieur,
- stérilisation en continu du produit en vrac à l'intérieur du tunnel,

B 7531 GP

- introduction en continu du produit stérile en vrac dans l'enceinte de traitement,

- traitement et manipulation du produit stérile dans l'enceinte de traitement,

- extraction du produit traité hors de l'enceinte de traitement, par passage en continu par un sas de sortie définissant une barrière fluide.

Grâce à ces caractéristiques, on conçoit qu'on réalise une chaîne stérile comprenant la stérilisation proprement dite, le stockage ou le traitement dans une enceinte de réception, et l'acheminement jusqu'à une enceinte d'utilisation n'appartenant pas à l'invention, sans nécessiter aucun emballage tout au long de la chaîne stérile.

Conformément à la première variante de réalisation de l'invention, il est également proposé une installation pour la stérilisation, l'introduction dans une enceinte de stockage biologiquement étanche et l'extraction d'un produit en vrac, caractérisée en ce qu'elle comprend :

- ladite enceinte de stockage biologiquement étanche,

- un circuit biologiquement étanche de ventilation et de filtration en continu d'un milieu contenu dans l'enceinte, incorporant des moyens pour stériliser initialement cette dernière,

- au moins une enceinte de stérilisation du produit à introduire en vrac dans l'enceinte de stockage, cette enceinte de stérilisation comprenant une porte ouvrant vers l'extérieur, une autre porte ouvrant directement dans l'enceinte de stockage et des moyens pour stériliser ledit produit,

- au moins un conteneur de transfert apte à être accouplé à l'enceinte de stockage au moyen d'un dispositif d'accouplement à double porte assurant le transfert étanche dudit produit entre l'enceinte de

B 7531 GP

stockage et le conteneur lorsque la double porte est retirée, et

- des moyens de manipulation dudit produit à l'intérieur de l'enceinte de stockage.

Dans ce cas, les moyens pour stériliser le produit dans l'enceinte de stérilisation peuvent être soit des moyens thermiques, soit des moyens chimiques, soit des moyens par rayonnement.

Conformément à la deuxième variante de réalisation de l'invention, il est également proposé une installation pour la stérilisation, l'introduction dans une enceinte de traitement biologiquement étanche et l'extraction en continu d'un produit en vrac, caractérisée en ce qu'elle comprend :

- ladite enceinte de traitement biologiquement étanche,

- un circuit biologiquement étanche de ventilation et de filtration en continu d'un milieu contenu dans l'enceinte, incorporant des moyens pour stériliser initialement cette dernière,

- au moins une enceinte de stérilisation du produit à introduire en vrac dans l'enceinte de traitement, cette enceinte de stérilisation comprenant une extrémité ouvrant vers l'extérieur, une autre extrémité débouchant dans l'enceinte de traitement et comportant une porte, et des moyens pour stériliser ledit produit,

- au moins un sas de sortie communiquant avec l'enceinte de traitement et débouchant à l'extérieur, ce sas comprenant une barrière fluide séparant l'air contenu dans l'enceinte de traitement de l'extérieur, et

- des moyens de traitement et de manipulation du produit à l'intérieur de l'enceinte de traitement.

Dans ce cas, les moyens de stérilisation sont, de préférence, des moyens thermiques placés en amont de ladite porte, un premier convoyeur étant placé entre le passage d'entrée et ladite porte, et un deuxième convoyeur étant placé entre la porte et l'enceinte de traitement.

On décrira maintenant, à titre d'exemple non limitatif, différentes variantes de réalisation de l'invention en se référant aux dessins annexés dans lesquels :

- la figure 1, déjà décrite, illustre de façon schématique une installation de la technique antérieure dans laquelle un produit emballé est stérilisé dans une première enceinte, avant d'être transporté dans son emballage jusqu'à une enceinte de réception, dans laquelle il pénètre après une nouvelle stérilisation de l'extérieur de l'emballage,

- la figure 2 illustre de façon schématique une installation selon l'invention dans laquelle un produit est stérilisé de façon discontinue et en vrac dans une enceinte de stérilisation étanche ouvrant directement dans l'enceinte de stockage, et

- la figure 3 illustre de façon schématique une variante de l'installation de la figure 2 dans laquelle la stérilisation s'effectue en continu à l'intérieur d'un tunnel ouvrant directement dans l'enceinte de traitement.

Comme l'illustre la figure 1 et comme on l'a déjà indiqué précédemment, conformément à la technique antérieure, lorsqu'un produit P doit être introduit dans une enceinte de réception étanche ER, ce produit doit d'abord être stérilisé à l'intérieur d'un emballage E dans une enceinte de stérilisation thermique ST externe à l'enceinte ER. Lorsque la stérilisation est terminée, l'enceinte de stérilisation est ouverte et le produit P emballé est transporté dans l'air ambiant jusqu'à l'enceinte de réception ER. A ce niveau, on a vu qu'il est nécessaire de stériliser à nouveau l'extérieur de l'emballage E à l'intérieur d'une enceinte de stérilisation par voie chimique SC ouvrant dans l'enceinte de réception.

Ces différentes opérations sont évidemment relativement longues et elles nécessitent un matériel coûteux. De plus, elles interdisent toute manipulation en vrac des produits et nécessitent l'utilisation systématique d'emballages.

Conformément à l'invention, il est proposé de remédier à ces inconvénients en utilisant un procédé et une installation de stérilisation, d'introduction d'un produit dans un isolateur et d'extraction de ce produit, de conception particulièrement simple, assurant une continuité parfaite de la stérilité des produits et permettant de stériliser, d'introduire et d'extraire ceux-ci en vrac, c'est-à-dire sans emballage. Bien entendu, le terme "produit" désigne ici aussi bien les produits que les matériels.

On décrira maintenant en se référant à la figure 2 une application de l'invention au cas où l'atmosphère contenue dans l'isolateur, ou enceinte de réception et de stockage biologiquement étanche 10 est une atmosphère stérile. On maintient en permanence dans cette enceinte 10 une atmosphère stérile ventilée, et en surpression à l'aide d'un circuit 12 représenté schématiquement sur la figure et qui comprend principalement une canalisation d'entrée 18 dans laquelle sont disposées en série et dans cet ordre un ventilateur de soufflage 24, une vanne à passage intégral 22 et une batterie de filtres 20 placés à l'entrée de l'enceinte 10, et une canalisation de sortie 14 dans laquelle est disposée une batterie de filtres

B 7531 GP

16. Le ventilateur 24 sert à mettre en surpression le volume clos stérile. A cet effet, il souffle l'air dans ce volume. La vanne 22 sert à réguler la surpression interne.

La stérilisation de l'enceinte 10 peut être effectuée par tout moyen approprié et notamment en plaçant un stérilisateur chimique (à vapeur saturante de formol ou d'acide paracétique) en dérivation sur la canalisation d'entrée 18, en amont des filtres 20. Une gaine d'extraction (non représentée) placée en aval des filtres 16 évite aux vapeurs résiduelles sortant de l'enceinte 10 lors de la stérilisation d'incommoder le personnel présent dans le bâtiment.

De préférence, les filtres 16 et 20 sont des filtres de type absolu qui sont réalisés en papier de verre et montés à l'intérieur d'un carter étanche métallique ou plastique. Chaque filtre présente une efficacité de 99,99% pour des particules de 0,3 μ. Ces caractéristiques permettent de maintenir le volume clos 10 en atmosphère stérile. Elles permettent aussi de protéger l'enceinte vis-à-vis des contaminants contenus dans l'atmosphère externe.

A titre d'exemple, le circuit de ventilation-filtration 12 peut être calculé afin de maintenir une surpression interne de 3 mm de colonne d'eau pour un débit d'air frais de 100 m$^3$/heure. L'énergie nécessaire est alors très faible et ne nécessite qu'un moteur de 60 Watts pour le ventilateur 24.

Conformément à une caractéristique essentielle de l'invention, l'installation comporte en outre au moins une enceinte de stérilisation 26 munie d'une porte d'entrée 28 ouvrant vers l'extérieur et d'une porte de sortie 30 ouvrant directement à l'intérieur de l'enceinte de réception 10.

De façon plus précise, la paroi de l'encein-

B 7531 GP

te de stérilisation 26 est raccordée de façon étanche à la paroi de l'enceinte 10 et les portes 28 et 30 assurent la fermeture étanche de l'enceinte 26, lorsqu'elles sont fermées.

Grâce à ces caractéristiques essentielles, on comprend que l'isolateur 10 peut être stérilisé isolément après fermeture de la porte 30. Lorsqu'on désire introduire des produits P dans l'isolateur stérile, ces produits peuvent être introduits en vrac dans l'enceinte 26 par la porte d'entrée 28. Cette porte 28 est ensuite refermée (la porte 30 étant toujours fermée) et les produits en vrac sont stérilisés à l'intérieur de l'enceinte 26 à l'aide de moyens de stérilisation appropriés associés à cette dernière. Lorsque la stérilisation est terminée, on peut ouvrir la porte inter-enceintes 30 et transférer directement les produits en vrac dans l'enceinte de réception 10.

Conformément à l'invention, les moyens de stérilisation associés à l'enceinte 26 peuvent être de tout type connu afin de permettre l'introduction dans l'isolateur de tout type de produits ou d'appareils, quelles que soient leur nature et leur présentation, sans nécessiter de précautions particulières. Bien entendu, lorsque des produits nécessitant une stérilisation d'un type différent doivent être introduits dans l'isolateur, on prévoit une enceinte de stérilisation adaptée à chaque type de produit.

Ainsi, les moyens de stérilisation peuvent être thermiques, l'enceinte 26 étant alors soit un autoclave vapeur à deux portes, soit un four du type Pasteur. Les moyens de stérilisation peuvent aussi être des moyens chimiques, et l'enceinte 26 constitue alors un sas à acide péracétique, à formol, à oxyde d'éthylène ou à tout autre produit stérilisant. On peut aussi stériliser les produits P en vrac à l'aide de moyens de stérilisation par rayonnements γ ou ultra violets ou en utilisant des accélérateurs linéaires.

B 7531 GP

0095971

Le choix des moyens de stérilisation sera fait de manière que, pour chaque type de produit ou d'appareil à introduire dans l'enceinte, la stérilisation soit tout à fait efficace sans pour autant être destructive. Compte tenu notamment de ce second impératif, on utilisera par exemple un autoclave vapeur pour stériliser le matériel métallique, l'eau, le linge, ou encore la vaisselle, ainsi que pour stériliser de la nourriture emballée. En revanche, le papier, les médicaments, ainsi que la nourriture non emballée seront stérilisés par voie chimique ou par rayonnements.

Conformément à un autre aspect de l'invention, des moyens de manipulation sont prévus dans la cellule 10 afin de permettre la manipulation des produits ou des appareils sans rompre l'étanchéité de l'enceinte. Bien entendu, ces moyens sont adaptés à la fois aux dimensions de l'enceinte de réception 10 et aux différentes manipulations susceptibles de devoir être effectuées dans celle-ci. Ces moyens de manipulation peuvent être simples ou multiples et ils sont constitués par tout moyen connu de manipulation à distance. Par exemple, on a représenté sur la figure 2 un gant de manipulation 32 monté de façon étanche sur la paroi de la cellule 10, un demi-scaphandre 34 également monté de façon étanche sur la paroi de la cellule 10 et un scaphandre 36 permettant à une personne de rentrer complètement dans la cellule sans en rompre l'étanchéité. Bien entendu, on pourrait utiliser aussi tout autre moyen connu tel que des télémanipulateurs, et il va de soi que ces différents moyens peuvent être combinés si cela est nécessaire.

Conformément à encore un autre aspect important de l'invention, l'installation est complétée par la présence de moyens permettant d'extraire les produits de l'isolateur pour les transférer jusqu'à une

B 7531 GP

0095971

autre enceinte stérile pouvant se trouver à une distance quelconque de l'installation, sans rompre la continuité de l'atmosphère stérile environnant le produit. A cet effet, on voit sur la figure 2 que des moyens sont prévus sur la paroi de l'enceinte 10 pour permettre l'accostage d'au moins un conteneur de transfert 38 et le transfert du produit P depuis l'enceinte 10 jusqu'au conteneur 38 sans rompre l'étanchéité de l'installation ni le caractère stérile du volume clos. A cet effet, le transfert entre l'enceinte 10 et le conteneur 38 s'effectue par un système de double porte de transfert étanche 40 de type connu, tel que le système décrit et revendiqué dans le brevet français n° 71 17358 déposé le 13 mai 1971 par la Société La Calhène.

Il est à noter que la stérilisation des conteneurs 38 s'effectue simultanément à celle de l'enceinte 10, après enlèvement de la double porte 40.

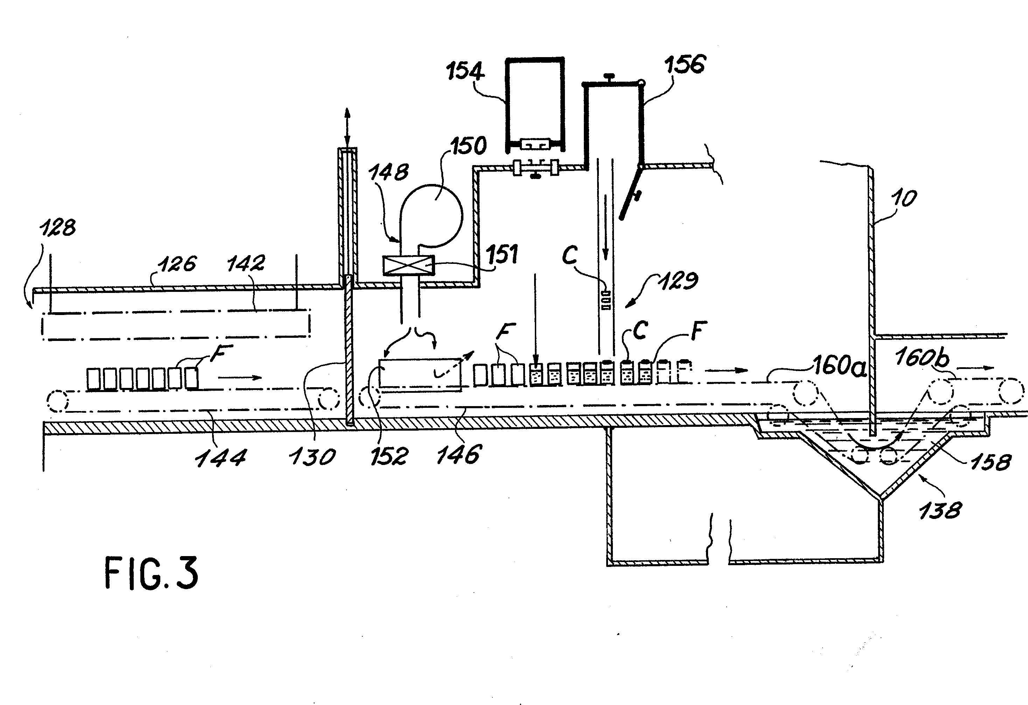

On a représenté sur la figure 3 une variante de l'installation de la figure 2 illustrant à titre d'exemple non limitatif un exemple d'application de l'installation selon l'invention. On comprendra que l'installation de la figure 3 peut aussi être combinée avec celle de la figure 2.

Comme l'installation de la figure 2, l'installation de la figure 3 permet conformément à l'invention de stériliser et d'introduire de manière simple et en vrac des produits ou des appareils dans une enceinte de réception, et de transférer ensuite les produits stériles hors de cette enceinte lorsque cela est nécessaire. Toutefois, l'installation de la figure 3 diffère de celle de la figure 2 par le caractère continu de la stérilisation et du transfert des produits à l'intérieur de l'enceinte de réception et par le caractère continu du transfert des produits stéri-

B 7531 GP

0095971

les hors de cette enceinte.

Ainsi, l'installation de la figure 3 comprend une enceinte de réception et de traitement biologiquement étanche et stérile 10 équipée comme celle de la figure 2 de moyens de ventilation et de filtration en continu (non représentés), de moyens de stérilisation (non représentés) et de moyens de manipulation (non représentés).

Dans l'installation de la figure 3, la stérilisation et l'introduction dans l'enceinte 10 du produit (constitué ici par des flacons F) s'effectuent en continu par un tunnel de stérilisation 126 dont une extrémité d'entrée 128 ouvre directement sur l'extérieur et dont l'extrémité de sortie 129 ouvre directement dans l'enceinte 10. Dans sa partie intermédiaire, le tunnel 126 peut être obturé par une porte 130 jouant comme on le verra ultérieurement un rôle différent de celui de la porte 30 dans l'installation de la figure 2. Dans la variante représentée, la porte 130 est une porte du type guillotine.

La partie du tunnel 126 située en amont de la porte 130 est équipée de moyens de stérilisation thermiques qui sont constitués d'une rampe de chauffage 142 disposée à la partie supérieure du tunnel. Les flacons à stériliser cheminent en continu dans cette première partie du tunnel 126 sur un premier convoyeur 144. Bien entendu, la vitesse d'avance du convoyeur 144 ainsi que la courbe de chauffage obtenue grâce à la rampe 142 sont conçues de façon à garantir une parfaite stérilisation de flacons F lorsqu'ils parviennent à proximité de la porte 130. A cet effet, les flacons doivent rester pendant 10 à 12 min. à une température proche de 300°C et sous une pression d'environ 5 mm de colonne d'eau.

En aval de la porte 130, le tunnel 126 est

B 7531 GP

0095971

muni d'un second convoyeur 146 permettant d'acheminer en continu les flacons F stérilisés jusqu'à l'intérieur de la cellule 10. Juste en aval de la porte 130, le tunnel 126 est muni de moyens de refroidissement 148 utilisant de l'air stérile circulant en circuit fermé. L'air stérile froid est refoulé par un ventilateur 150 à la partie supérieure du tunnel, au travers d'une batterie de filtres 151, pour venir refroidir les flacons F avant d'être repris par des orifices latéraux tels que 152 et aspiré à nouveau par le ventilateur 150.

Ensuite, dans la variante représentée, on peut prévoir directement à l'intérieur de la cellule 10, des moyens de remplissage et d'obturation des flacons F. Comme on l'a représenté à titre d'exemple sur la figure 3, les moyens de remplissage peuvent comprendre un conteneur de transfert stérile 154 accouplé à l'enceinte 10 au moyen d'un système de raccordement à double porte (comparables au conteneur 38 et à la double porte 40 sur la figure 2) par lequel on introduit le produit de remplissage. Bien entendu, l'introduction du produit dans les flacons peut être réalisée soit de façon automatique à l'aide d'un appareil de remplissage de type connu, soit manuellement à l'aide de moyens de manipulation à distance également de type connu. Lorsque le flacon est rempli, il est ensuite obturé par des bouchons et capsules C introduits et stérilisés dans une enceinte 156, les bouchons et capsules C pouvant ici encore être mis en place soit de façon automatique, soit à l'aide de moyens de manipulation à distance. De préférence, l'enceinte 156 est constituée par un autoclave de stérilisation comparable à l'autoclave 26 sur la figure 2.

Lorsque les flacons sont ainsi remplis et obturés, ils peuvent être soit stockés dans l'enceinte

B 7531 GP

0095971

10 pendant une période quelconque, soit transférés directement à l'extérieur de cette enceinte. Dans les deux cas, on peut prévoir des moyens de transfert en continu vers l'extérieur de l'enceinte. Dans la variante représentée, ces moyens sont constitués par un sas liquide 138 remplissant en continu la même fonction que le conteneur 38 remplit de façon discontinue dans le mode de réalisation de la figure 2. Ce sas liquide 138 est rempli d'un liquide bactéricide tel qu'une solution d'eau de javel 158 et il est équipé de préférence d'un convoyeur permettant d'acheminer les flacons à l'extérieur de l'enceinte 10 sans rompre l'étanchéité de celle-ci. A cet effet, le convoyeur est réalisé en deux parties 160a et 160b disposées respectivement à l'intérieur de la cellule 10 et à l'extérieur de cette cellule. Il est à noter qu'en plus d'assurer le maintien et la stérilité de l'atmosphère contenue dans l'enceinte 10, le sas liquide 138 permet également de laver les flacons lorsqu'ils sortent de l'enceinte. Les flacons peuvent ensuite être séchés par un moyen approprié (non représenté).

L'installation qui vient d'être décrite pourrait être complétée en prévoyant en amont du tunnel 126 ou à l'entrée 128 de celui-ci un poste de lavage des flacons F.

Dans l'installation qui vient d'être décrite en se référant à la figure 3, la stérilisation de l'enceinte 10 s'effectue après fermeture de la porte 130 à l'aide des moyens de stérilisation associés à l'enceinte 10. Lorsque cette stérilisation est terminée et lorsqu'on désire mettre en route l'installation, les moyens de chauffage 142 sont mis en oeuvre pour atteindre leur température de fonctionnement et c'est seulement à ce moment que la porte 130 est ouverte. En effet, les moyens de chauffage 142 consti-

B 7531 GP

tuent alors une barrière stérile parfaitement efficace pour préserver l'étanchéité de l'enceinte 10. La porte 130 est maintenue ouverte pendant toute la durée du fonctionnement des moyens de stérilisation par chauffage 142 et elle est fermée dès qu'il est décidé d'arrêter ces derniers, afin de préserver le confinement de l'isolateur.

On comprend que l'installation qui vient d'être décrite peut s'appliquer à un grand nombre d'utilisations nécessitant une enceinte stérile et l'entrée et la sortie de produits et d'appareils dans cette enceinte. Ainsi, l'installation de remplissage de flacons décrite en se référant à la figure 3 est particulièrement adaptée à l'industrie pharmaceutique. De façon comparable, l'installation décrite en se référant à la figure 2 peut être utilisée dans les hôpitaux ainsi que dans les laboratoires nécessitant une animalerie.

Les applications décrites en se référant aux figures 2 et 3 concernent le cas où l'atmosphère contenue dans l'isolateur est stérile. Toutefois, l'invention peut aussi s'appliquer au cas d'un isolateur contenant un milieu pathogène (laboratoires bactériologiques, fabrication de bactéries ou virus à haute pathogénicité, etc...), le circuit de ventilation filtration étant alors inversé afin de maintenir en permanence une dépression à l'intérieur de l'enceinte. L'installation est alors du même type que celle qui a été décrite en se référant à la figure 2 et peut être utilisée en mettant en oeuvre le même procédé. L'extraction du produit peut alors se faire soit par le conteneur 38 lorsqu'il s'agit de transférer les produits jusqu'à une autre enceinte étanche, soit par l'enceinte de stérilisation 26, lorsque les produits doivent être définitivement extraits de l'enceinte

B 7531 GP

0095971

contenant le milieu pathogène. Dans ce dernier cas, les produits sont à nouveau stérilisés dans l'enceinte 26 avant d'être sortis, de façon à éviter toute contamination de l'environnement par les virus ou bactéries contenus dans l'enceinte.

—

B 7531 GP

REVENDICATIONS

1. Procédé pour la stérilisation, l'introduction dans une enceinte de stockage biologiquement étanche et l'extraction d'un produit (P, F) en vrac, caractérisé en ce qu'il comprend les étapes successives suivantes :

- stérilisation initiale de l'enceinte de stockage (10),

- introduction du produit en vrac dans une enceinte de stérilisation (26) par une porte (28) ouvrant sur l'extérieur, ladite enceinte de stérilisation communiquant avec l'enceinte de stockage par une autre porte (30),

- fermeture des portes (28, 30) de l'enceinte de stérilisation,

- stérilisation du produit en vrac dans l'enceinte de stérilisation,

- ouverture de la porte (30) par laquelle l'enceinte de stérilisation communique avec l'enceinte de stockage,

- introduction du produit stérile directement dans l'enceinte de stockage,

- stockage et manipulation du produit dans l'enceinte de stockage,

- extraction du produit en vrac hors de l'enceinte de stockage, en introduisant ce produit dans au moins un conteneur de transfert (38) communiquant avec l'enceinte de stockage par un système de transfert à double porte (40), après ouverture de la double porte, en fermant la double porte et en déconnectant le conteneur de l'enceinte de stockage.

2. Procédé selon la revendication 1, caractérisé en ce qu'on transfère ensuite le produit dans

B 7531 GP

le conteneur (38) jusqu'à une enceinte de réception et en ce qu'on introduit le produit en vrac dans cette dernière par un autre système de transfert à double porte.

3. Procédé selon la revendication 1, caractérisé en ce qu'on stérilise le conteneur (38) simultanément à la stérilisation de l'enceinte de stockage, après ouverture de la double porte.

4. Installation pour la stérilisation, l'introduction dans une enceinte de stockage biologiquement étanche et l'extraction d'un produit en vrac, caractérisée en ce qu'elle comprend :
- ladite enceinte de stockage biologiquement étanche (10),
- un circuit biologiquement étanche (12) de ventilation et de filtration en continu d'un milieu contenu dans l'enceinte, incorporant des moyens pour stériliser initialement cette dernière,
- au moins une enceinte de stérilisation (26) du produit à introduire en vrac dans l'enceinte de stockage, cette enceinte de stérilisation comprenant une porte (28) ouvrant vers l'extérieur, une autre porte (30) ouvrant directement dans l'enceinte de stockage et des moyens pour stériliser ledit produit,
- au moins un conteneur de transfert (38) apte à être accouplé à l'enceinte de stockage au moyen d'un dispositif d'accouplement à double porte (40) assurant le transfert étanche dudit produit entre l'enceinte de stockage et le conteneur lorsque la double porte est retirée, et
- des moyens de manipulation (32 à 36) dudit produit à l'intérieur de l'enceinte de stockage.

5. Installation selon la revendication 4, caractérisée en ce que les moyens pour stériliser le produit dans l'enceinte de stérilisation sont des moyens thermiques.

B 7531 GP

6. Installation selon la revendication 4, caractérisée en ce que les moyens pour stériliser le produit dans l'enceinte de stérilisation sont des moyens chimiques.

7. Installation selon la revendication 4, caractérisée en ce que les moyens pour stériliser le produit dans l'enceinte de stérilisation sont des moyens par rayonnements.

8. Procédé pour la stérilisation, l'introduction dans une enceinte de traitement biologiquement étanche et l'extraction en continu d'un produit en vrac, caractérisé en ce qu'il comprend les étapes successives suivantes :

- stérilisation initiale de l'enceinte de traitement (10) après fermeture d'une porte (130) par laquelle cette dernière communique avec une enceinte de stérilisation (126) en forme de tunnel,
- mise en route de moyens de stérilisation à l'intérieur du tunnel,
- ouverture de ladite porte (130),
- introduction en continu du produit en vrac par une extrémité du tunnel ouvrant sur l'extérieur,
- stérilisation en continu du produit en vrac à l'intérieur du tunnel,
- introduction en continu du produit stérile en vrac dans l'enceinte de traitement,
- traitement et manipulation du produit stérile dans l'enceinte de traitement,
- extraction du produit traité hors de l'enceinte de traitement, par passage en continu par un sas de sortie (138) définissant une barrière fluide.

9. Installation pour la stérilisation, l'introduction dans une enceinte de traitement biologiquement étanche et l'extraction en continu d'un produit en vrac, caractérisée en ce qu'elle comprend :

B 7531 GP

- ladite enceinte de traitement biologiquement étanche (10),
- un circuit biologiquement étanche (12) de ventilation et de filtration en continu d'un milieu contenu dans l'enceinte, incorporant des moyens pour stériliser initialement cette dernière,
- au moins une enceinte de stérilisation (126) du produit à introduire en vrac dans l'enceinte de traitement, cette enceinte de stérilisation comprenant une extrémité (128) ouvrant vers l'extérieur, une autre extrémité débouchant dans l'enceinte de traitement et comportant une porte (130), et des moyens pour stériliser ledit produit,
- au moins un sas de sortie (138) communiquant avec l'enceinte de traitement et débouchant à l'extérieur, ce sas comprenant une barrière fluide séparant l'air contenu dans l'enceinte de traitement de l'extérieur, et
- des moyens de traitement et de manipulation (129) du produit à l'intérieur de l'enceinte de traitement.

10. Installation selon la revendication 9, caractérisée en ce que les moyens de stérilisation sont des moyens thermiques (142) placés en amont de ladite porte, un premier convoyeur (144) étant placé entre le passage d'entrée et ladite porte (130) et un deuxième convoyeur (146) étant placé entre la porte et l'enceinte de traitement.

11. Installation selon la revendication 9, caractérisée en ce que le sas de sortie comprend une barrière liquide (158).

B 7531 GP

FIG.1

FIG.2

FIG.3

0095971

RAPPORT DE RECHERCHE EUROPEENNE

Office européen
des brevets

Numéro de la demande

EP 83 40 1014

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | FR-A-2 084 037 (UNILEVER)<br><br>* Page 1, ligne 8 - page 5, ligne 36 * | 1,4,6, 8,9 | A 61 L 2/20<br>A 61 L 2/06 |
| Y | US-A-3 035 886 (FRANK D. HICKEY)<br>* Colonne 1, ligne 55 - colonne 2, ligne 45; colonne 3, ligne 29 - colonne 7, ligne 17 * | 1 | |
| Y | DE-A-1 251 469 (KARL OSKAR LENNART SILVERSTOLPE)<br>* Colonne 2, ligne 37 - colonne 4, ligne 21 * | 1,4,7 | |
| A | DE-C- 929 574 (DR. MAX ERICH SCHULZ)<br>* En entier * | | |
| A | CH-A- 443 569 (FRANCOIS DESHUSSES)<br>* Colonne 2, ligne 14 - colonne 8, ligne 4 * | | |
| A | CH-A- 345 260 (RAYMOND JOVIGNOT)<br>* Page 2, ligne 54 - page 4, ligne 2 * | | |

DOMAINES TECHNIQUES RECHERCHES (Int. Cl. 3)

A 61 L

--- -/-

Le présent rapport de recherche a été établi pour toutes les revendications

| Lieu de la recherche | Date d'achèvement de la recherche | Examinateur |
|---|---|---|
| LA HAYE | 30-08-1983 | SCHRIJVERS H.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 .13 82

Office européen
des brevets

**RAPPORT DE RECHERCHE EUROPEENNE**

| | | Page 2 |
|---|---|---|

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| A | GB-A-2 028 658 (VICKERS) <br><br> * En entier * <br><br> ----- | | |

**DOMAINES TECHNIQUES RECHERCHES (Int. Cl. ³)**

Le présent rapport de recherche a été établi pour toutes les revendications

| Lieu de la recherche<br>LA HAYE | Date d'achèvement de la recherche<br>30-08-1983 | Examinateur<br>SCHRIJVERS H.J. |
|---|---|---|

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

& : membre de la même famille, document correspondant

OEB Form 1503. 03.82